# NOUVEAU FASCICULE DE BREVET EUROPEEN

(11) **EP 1 015 579 B2**
(45) Date de publication et mention de la décision concernant l'opposition: **06.12.2017**
(45) Mention de la délivrance du brevet: 16.04.2014
(21) Numéro de dépôt: 98944017.7
(22) Date de dépôt: 17.09.1998
(51) Int. Cl.: C12N 15/11, C12N 15/31, C12N 1/21, C07K 14/33, A61K 39/08

(54) **TOXINE DE CLOSTRIDIUM ET PROCEDE DE PREPARATION DE COMPOSITIONS IMMUNOGENES**
CLOSTRIDIUMTOXIN UND VERFAHREN ZUR HERSTELLUNG IMMUNOGENE ZUSAMMENSETZUNGEN
CLOSTRIDIUM TOXIN, AND METHOD FOR PREPARING IMMUNOGENIC COMPOSITIONS

(30) Priorité: 19.09.1997 FR 9711710
(43) Date de publication de la demande: 05.07.2000
(73) Titulaire: INSTITUT PASTEUR, 75724 Paris Cedex 15 (FR)
(72) Inventeur: GIBERT, Maryse, F-92160 Antony (FR); POPOFF, Michel-Robert, F-92140 Clamart (FR)
(74) Mandataire: D Young & Co LLP
(86) Numéro de dépôt international: PCT/FR1998/001999
(87) Numéro de publication internationale: WO 1999/015669

(56) Documents cités:
- EP-A- 0 453 216
- WO-A-95/17521
- WO-A-97/34001
- HUNTER S E C ET AL: "CLONING AND NUCLEOTIDE SEQUENCING OF THE CLOSTRIDIUM PERFRINGENS EPSILON-TOXIN GENE AND ITS EXPRESSION IN ESCHERICHIA COLI" INFECTION AND IMMUNITY, vol. 60, no. 1, janvier 1992, pages 102-110, XP000674523
- BULLIFENT H L ET AL: "The construction of a reporter system and use for the investigation of Clostridium perfringens gene expression." FEMS MICROBIOLOGY LETTERS, vol. 131, no. 1, - 15 août 1995 pages 99-105, XP002067231
- GIBERT M ET AL: "Beta2 toxin, a novel toxin produced by Clostridium perfringens [published erratum appears in Gene 1998 Mar 27;210(1):173]." GENE, (1997 DEC 5) 203 (1) 65-73. JOURNAL CODE: FOP. ISSN: 0378-1119., XP002099309 Netherlands

## Description

La présente invention concerne de nouveaux acides nucléiques codant pour la toxine β₂ mature et sa séquence d'adressage de Clostridium perfringens. Elle concerne également un procédé de préparation de polypeptides recombinants utilisant ces acides nucléiques, ainsi que les cellules recombinantes contenant ces acides nucléiques. La demande décrit en outre un nouveau procédé de préparation d'antigènes ou fragments d'antigènes, notamment de toxines bactériennes, plus préférentiellement de toxines de *Clostridium,* en vue de la préparation de compositions immunogènes et/ou vaccinales. Elle décrit encore des compositions immunogènes et/ou vaccinales ayant des propriétés améliorées.

La présente invention concerne plus particulièrement le domaine de la production de protéines toxiques bactériennes, notamment de toxines de *Clostridium* ou d'autres organismes pathogènes. Elle concerne en particulier la production améliorée de ces toxines, dans le but de réaliser des préparations immunogènes ayant un pouvoir vaccinant accru.

Les pathologies d'origine bactérienne (choléra, dysentrie, entérites, etc) sont une cause de mortalité importante chez l'homme comme chez l'animal. Ces pathologies sont essentiellement d'origine alimentaire, liées à la présence dans les éléments ingérés de bactéries pathogènes qui vont coloniser les parois mucosales, puis engendrer une toxicité et une nécrose des tissus. Les bactéries pathogènes appartiennent à des genres différents, parmi lesquels on peut citer notamment Actinomycetes, Bacillus, Bordetella, Clamydia, Clostridium, Corynebacterium, Escherichia, Fusobacterium, Listeria, Mycobacterium, Mycoplasma, Samonella, Staphylococcus, Treponemo ou encore Vibro. Parmi ces bactéries pathogènes, les bactéries Clostridium représentent une classe importante, dont on peut mentionner par exemple les espèces suivantes : C. absonum, C. baratii, C. bifermentans, C. chauvei, C. difficile, C. ghonii, C. lituseburense, C. novyi, C. perfringens, C. septicum, C. sordellii, C. subterminale et C. tetani.

Il a été montré que la pathogénicité de ces bactéries pathogènes est liée à la production, par celles-ci, de toxines ou entérotoxines. Aujourd'hui, la grande majorité de ces toxines a été identifiée et caractérisée.

Ainsi, la pathogénicité des souches de Clostridium septicum, connues comme étant responsables de la gangrène atraumatique, est liée à l'expression d'un facteur létal unique, désigné toxine alpha, dont le gène a été cloné (Ballard et al., Infection and Immunity 63 (1995) 340). La toxine produite par les souches de Clostridium sordellii a été désignée cytotoxine Cs Cyt. Les souches de C. perfringens sont généralement classées en 5 types (A-E) en fonction de la nature des toxines qu'elles produisent (toxines alpha, béta, epsilon, iota et entérotoxine). La toxicité de C. tetanii est liée à la production d'une toxine, de même que Bordetella produit la toxine pertussique ou C. tetani la toxine tétanique. D'autres toxines sont indiquées dans la suite du texte.

Les traitements disponibles actuellement sont essentiellement d'ordre prophylactique. Ainsi, des préparations vaccinales sont produites à partir de cultures de souches de bactéries pathogènes. Les surnageants contenant les toxines sont ensuite récoltés puis soumis à différentes étapes de concentration et/ou purification partielle. Les surnageants ou leurs filtrats/concentrats sont ensuite soumis à une étape d'inactivation, afin de produire des toxines non-virulentes, mais conservant leur pouvoir immunogène (toxoïdes). Différentes techniques sont disponibles pour inactiver les toxines, et notamment des techniques chimiques ou génétiques, qui seront détaillées plus loin. Par ailleurs, le plus souvent, préalablement à l'étape d'inactivation, les surnageants de cultures de différents organismes pathogènes sont regroupés afin d'obtenir des cocktails de toxines, dans le but de préparer des vaccins polyvalents.

Des exemples de tels vaccins disponibles dans le commerce sont par exemple le Miloxan^{R}, commercialisé par la société Rhône-Mérieux (Mérial), France, permettant une protection des animaux contre les toxi-infections et les entérotoxémies dues à des souches de *Clostridium perfringens* et de *Clostridium sordellii.* Plus particulièrement, cette préparation vaccinale contient des toxoïdes de *Clostridium perfringens* types B, C et D, de *Clostridium septicum,* de *Clostridium* novyi de *Clostridium tetani,* de *Clostridium chauvei* et de *Clostridium sordellii.* Un autre vaccin polyvalent est par exemple le Gletvax5^{R}, commercialisé par la société Mallinckrodt Veterinary, France, permettant une protection contre la collibacilose des porcelets et les entérites à *Clostridium perfringens* de type C. Ce vaccin contient plus particulièrement des antigènes de E. coli, ainsi que des toxoïdes de la toxine béta de *Clostridium perfringens* type C.

Les vaccins disponibles aujourd'hui présentent toutefois un certain nombres d'inconvénients. Ainsi, il s'agit essentiellement de mélanges de surnageants de cultures, dont la composition n'est pas définie exactement et dont la reproductibilité n'est donc pas entièrement assurée. En outre, s'agissant de vaccins polyvalents protégeant contre des organismes pathogènes différents, leur production implique des fermentations séparées, d'organismes différents, et donc des conditions de culture et des normes de sécurité très contraignantes sur le plan industriel. En outre, certains de ces vaccins ont une efficacité limitée à l'égard de certaines toxines, notamment de celles produites en quantités faibles.par les organismes pathogènes.

Il existe donc un réel besoin de pouvoir améliorer les conditions de préparation, la qualité et l'efficacité des vaccins contre les toxines bactériennes. La présente invention apporte une solution avantageuse à ces problèmes.

La présente demande décrit en effet de nouveaux acides nucléiques permettant l'expression de transgènes dans des bactéries, notamment de type Clostridium. La demande rapporte également des constructions permettant la production dans des bactéries, notamment de type Clostridium, de quantités plus importantes de toxines, dans un but vaccinal notamment. La présente demande décrit en particulier des souches de bactéries recombinantes, notamment de type Clostridium, permettant la production amplifiée de toxines, qu'il s'agisse de toxines de Clostridium ou de toxines d'autres organismes pathogènes. La demande divulgue également des souches de bactéries recombinantes, notamment de type Clostridium, permettant la production de plusieurs toxines simultanément.

La demande divulgue ainsi une méthode de production de toxines recombinantes permettant d'augmenter le caractère immunogène des préparations vaccinantes, et ainsi leur effet protecteur.

La demande divulgue aussi la production par voie recombinante de nouvelles toxines, permettant d'élargir la gamme des vaccins existants, notamment vis-à-vis de la toxine béta2 de *Clostridium perfringens* ou d'autres toxines faiblement produites par les organismes pathogènes, ou faiblement immunogènes.

Les informations de la demande permettent également une mise en oeuvre industrielle considérablement plus aisée, dans la mesure où les volumes de surnageants produits et où la diversité des organismes producteurs utilisés peuvent être réduits significativement.

La présente invention porte sur un acide nucléique choisi dans le groupe constitué par :
a) un acide nucléique, codant pour la toxine β2 mature et sa séquence d'adressage de *Clostridium perfringens,* tel que défini à la SEQ ID NO :1 ; et
b) un acide nucléique, codant pour un variant de la toxine β2 mature et sa séquence d'adressage telle que définie à la SEQ ID NO :1, ladite toxine β2 mature du variant présentant des mutations de ses résidus d'acides aminés par rapport à la SEQ ID NO :1, ledit variant conservant la capacité à être sécrété.

Un aspect concerne des acides nucléiques et des constructions génétiques permettant une production améliorée de protéines chez les bactéries, notamment de toxines bactériennes dans les bactéries du genre *Clostridium,* particulièrement *Clostridium perfringens.*

Ainsi, le demandeur a isolé à partir du génome d'une souche de *Clostridium perfringens* de type C le gène complet codant pour une toxine, désignée toxine béta2 (SEQ ID n° 1). L'étude du gène obtenu a montré que ce gène comporte en outre, en 5', une région promotrice de la transcription (SEQ ID n° 2), efficace dans les bactéries notamment dans les bactéries du genre *Clostridium,* particulièrement *Clostridium perfringens.*

Un premier objet décrit dans la demande réside donc plus particulièrement dans un acide nucléique caractérisé en ce qu'il présente une activité de promoteur transcriptionnel et en ce qu'il comprend :
(a) tout ou une partie de la séquence SEQ ID n° 2 ou d'un variant de celle-ci, ou
(b) une séquence hybridant avec tout ou partie du brin complémentaire de la séquence SEQ ID n° 2.

Plus préférentiellement, cet acide nucléique comprend tout ou une partie de la séquence SEQ ID n° 2.

Avantageusement, cet acide nucléique est constitué du promoteur du gène de la toxine béta 2 de *Clostridium perfringens* ou d'un fragment de celui-ci.

Le terme "acide nucléique" désigne au sens de l'invention tout acide désoxyribonucléique (ADN) ou ribonucléique (ARN). Un ADN peut être plus particulièrement un ADN complémentaire (ADNc), un ADN génomique (ADNg) ou un ADN synthétique. Au sens de la présente invention, le terme "acide nucléique" est également synonyme de "polynucléotide". Les acides nucléiques décrits peuvent être d'origine diverses, et notamment d'origine bactérienne, synthétique ou semi-synthétique. Ils peuvent être isolés par toutes les techniques connues de la Biologie moléculaire, en utilisant les données structurales et de séquences fournies par la présente demande. Ainsi, ces acides nucléiques peuvent être isolés à partir de banques, par des techniques d'hybridation. Ils peuvent aussi être synthétisés chimiquement ou génétiquement.

Le terme "partie" ou "fragment" de l'acide nucléique désigne tout acide nucléique comportant au moins une partie de la séquence concernée (par exemple la séquence SEQ ID n° 2) et conservant une activité de promoteur transcriptionnel. La partie de la séquence comprend avantageusement 50 pb au moins, plus préférentiellement au moins 100 pb. Ces "parties" peuvent être générées aisément par les techniques classiques de la biologie moléculaire, soit par clivage et digestion enzymatiques à partir des fragments décrits, soit par synthèse en utilisant des synthétiseurs d'acides nucléiques.

Le terme "hybridant" désigne au sens de l'invention toute hybridation dans des conditions normales, stringentes ou non, telles que définies ci-après. Des conditions d'hybridation stringentes sont par exemple : Hybridation à 42°C, 50% formamide, 5 X SSC, 1 X Denhardt ; Lavage à 65°C en 0,1 X SSC, 0,1% SDS. Des conditions non-stringentes sont notamment : Hybridation à 37°C, 40% formamide, 5 X SSC, 1 X Denhardt ; Lavage à 50°C en 1 X SSC, 0.1% SDS. Les conditions stringentes sont particulièrement adaptées lorsque les acides nucléiques sont présents en faibles quantités et/ou sous forme peu purifiée. Les conditions non stringentes sont plus adaptées lorsque l'acide nucléique est présent en quantités plus importantes et se trouve sous forme significativement représentée dans l'échantillon. Avantageusement, les séquences "hybridant" sont des séquences qui hybrident en conditions stringentes, et qui possèdent donc un degré d'homologie structurale élevé avec la séquence concernée (par exemple la séquence SEQ ID n° 2) ou ses fragments. En outre, les séquences hybridant peuvent comporter une région permettant l'hybridation et une région contigüe n'hybridant pas, mais correspondant aux régions flanquantes.

Par ailleurs, l'activité de promoteur transcriptionnel des "fragments" ou "parties" et "séquences hybridant" peut être contrôlée aisément par l'homme du métier selon la méthodologie décrite dans les exemples. En particulier, l'activité des fragments/hybridants peut être vérifiée en introduisant ces acides nucléiques en 5' d'un gène marqueur, puis en étudiant l'expression de ce marqueur dans une population de cellules telles que par exemple des bactéries du genre *Clostridium,* notamment *Clostridium perfringens.*

Les exemples présentés plus loin montrent que les acides nucléiques décrits dans la demande permettent d'amplifier de manière significative l'expression d'une protéine dans une bactérie, notamment dans un *Clostridium perfringens.* Ainsi, sur une souche sauvage productrice de toxine beta2, le niveau de production est augmenté d'un facteur 40 à 80 environ en présence d'un acide nucléique de l'invention. Par ailleurs, les exemples qui suivent montrent en outre que ces acides nucléiques permettent l'expression à des taux significatifs de protéines hétérologues dans les bactéries du genre Clostridium. En particulier, les exemples montrent que les acides nucléiques de l'invention permettent la production de toxines hétérologues dans ces bactéries.

A cet égard, la demande concerne également une cassette d'expression d'un transgène caractérisée en ce qu'elle comprend, dans l'orientation 5' -> 3' :
- un acide nucléique tel que défini ci-avant, et
- ledit transgène.

Dans la cassette, l'acide nucléique et le transgène sont liés ensemble de manière opérationnelle (c'est à dire de sorte que l'acide nucléique permet l'expression dudit transgène).

Avantageusement, la cassette comprend en outre, en 3' du transgène, un terminateur transcriptionnel.
Par ailleurs, la cassette peut également comporter de manière avantageuse un signal de sécrétion, permettant d'induire ou d'augmenter la sécrétion du produit d'expression du transgène par les cellules. Avantageusement, ce signal de sécrétion est localisé entre l'acide nucléique de l'invention et le transgène, en phase de lecture avec ce dernier.

A cet effet, les inventeurs ont également mis en évidence, dans le gène identifié, l'existence d'un tel signal de sécrétion, particulièrement actif dans les bactéries du genre Clostridium. Ce signal est représenté par les résidus 268-357 sur la séquence SEQ ID n° 1, et séparément sur la séquence SEQ ID n°3. Ce signal, ou tout variant ou fragment actif de celui-ci, constitue un mode de réalisation avantageux de l'invention.

Comme indiqué précédemment, l'invention est particulièrement appropriée pour la production de toxines ou variants de la toxine βB₂ de C. perfringens.

On entend par "toxine" au sens de l'invention tout peptide, polypeptide ou protéine produit par une bactérie pathogène, et impliqué dans ladite activité pathogène. Il peut s'agir d'un facteur directement responsable de la toxicité de la bactérie, ou participant à cette toxicité. Un "fragment" de toxine peut être constitué de toute partie d'une toxine, ayant conservé certains motifs immunogéniques de la toxine. En particulier, il a été décrit que les toxines bactériennes présentent souvent différents domaines fonctionnels distincts, et notamment un domaine impliqué dans l'activité toxique (site catalytique) distinct d'autres domaines impliqués dans la reconnaissance de sites ou dans des interactions avec des partenaires. Un "fragment" de toxine est constitué avantageusement d'un domaine dépourvu d'activité toxique, mais conservant un pouvoir immunogène. Un variant de toxine peut être constitué par exemple d'un dérivé résultant de modifications génétiques de la séquence codant pour ladite toxine ou fragment de toxine. De telles modifications génétiques sont par exemple des mutations, délétions, fusions, etc. Généralement, les mutations affectent de 1 à 10 résidus, de préférence de 1 à 5 résidus. Ces mutations sont des mutations modifiant l'acide aminé codé, et donc la séquence de la protéine. Les délétions peuvent être des délétions internes ou terminales. Elles peuvent effecter jusqu'à 40% de la séquence entière. Les fusions consistent à introduire des régions supplémentaires en 5' et/ou en 3' de la séquence, ou éventuellement à insérer de telles régions au sein de la séquence. Ces modifications peuvent être effectuées dans le but soit de diminuer, voire supprimer la toxicité de ces protéines, soit d'améliorer leur production ou leur stabilité par exemple. De telles modifications génétiques peuvent être réalisées selon les conditions classiques de la biologie moléculaire, et sont illustrées dans l'art antérieur ainsi que dans la suite du texte de la présente demande.

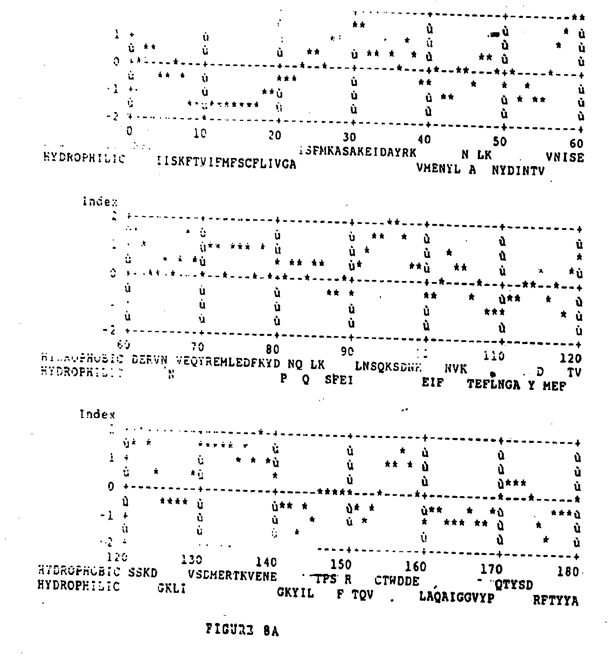

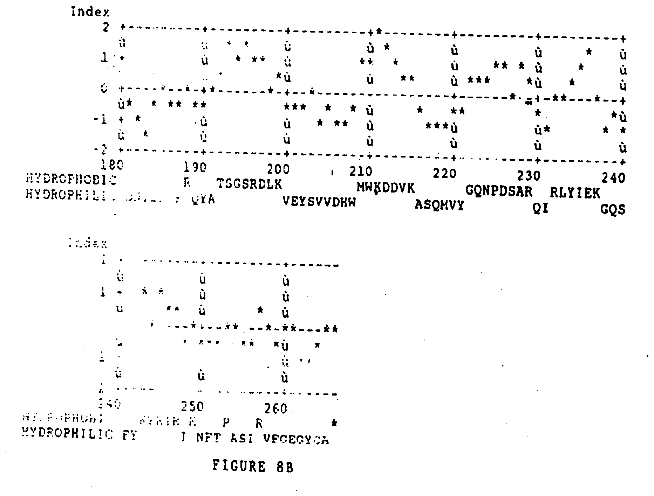

Plus particulièrement, les cassettes décrites dans la demande sont adaptées à la production des toxines ou variant suivants :
- Toxine Beta 2 de *Clostridium perfringens* ou tout fragment immunogène. Le profil d'hydrophilicité de la toxine Beta2 est représenté sur la figure 8. Ce profil fait apparaitre plusieurs régions hydrophiles, qui définissent des fragments particuliers au sens de l'invention. Ces régions sont notamment localisées au niveau des résidus d'acides aminés 40-55, 105-120, 160-170, 175-188, 200-210 et 250-260 tels que représentés sur la séquence SEQ ID n° 1. Par ailleurs, des fragments de la toxine Béta2 dépourvus de toxicité sont notamment les fragments trypsiques de 24, 15 et 13 kDa décrits dans les exemples. Des constructions pour l'expression de cette toxine sont décrites dans les exemples.
- Toxine Béta1 de *Clostridium perfringens* ou tout fragment immunogène. La séquence de cette toxine a été décrite dans la littérature (Hunter et al., Infect. Immun. 61 (1993) 3958-3965). Des constructions pour l'expression de cette toxine sont décrites dans les exemples.
- Toxines Iota de *Clostridium perfringens* ou tout fragment immunogène. La séquence des gènes codant pour les toxines lota1 (gène la) et lota2 (gène Ib) ont été décrites dans la littérature (Perelle et al., Infect. Immun. 61 (1993) 5147-5156).
- Toxine alpha de *C. novyi* ou tout fragment immunogène. La séquence du gène de cette toxine (gène tcnα) a été décrite dans la littérature (Hofmann et al., Mol. Gen. Genet. 247 (1995) 670-679).
- Toxine alpha de *C. septicum* ou tout fragment immunogène. La séquence du gène de cette toxine a été décrite dans la littérature (Ballard et al., Infect. Immun. 63 (1995) 340-344).
- Toxines A et B de *C. difficile*, ou tout fragment immunogène. La séquence du gène de ces toxines a été décrite dans la littérature, ainsi que différentes régions immunogènes (Von Eichel-Streiber et al., Mol. Gen. Genet. 233 (1992) 260-268 ; Von Eichel-Streiber et al., J. Gen. Microbio. 135 (1989) 55-64 ; Von Eichel-Streiber et al., J. Bacteriol. 174 (1992) 6707-6710).
- Toxine epsilon de *C. perfringens* (Worthington et al., Onderstepoort J. Vet. Res. 40 (4) (1973) 145-152; Hunter et al. Infect. Immun. 60, (1992) 102-110).
- Enterotoxine de *C. perfringens* (McClane, Toxicon. 34 (1996) 1335-1343).
- Toxine de *C. chauvoei* (Crichton et al., Australian Vet. J. 63 (1986) 68).
- Cytotoxine L de *C. sordellii* ou tout fragment immunogène. La séquence du gène de cette toxine a été décrite dans la littérature (Green et al., Gene 161 (1995) 57-61), ainsi que différentes régions immunogènes. En particulier, cette toxine est constituée d'une protéine de 270 kDa environ et différents fragments antigéniques ont été décrits.
- Toxine pertussis.
- Toxine tétanique, toxique botulique, en particulier le fragment C de ces toxines qui est le fragment immunogène (Makoff et al., Bio/Technology 7 (1989) 1043; Figueiredo et al., Infect. Immun. 63 (1995) 3218-3221; Wells et al., Molecular Microbiol. 8 (1993) 1155-1162; Boucher et al, Infect. Immun. 62 (1994) 449-456, Clare et al., Bio/Technology 9 (1991) 455; Clayton et al., Infect. Immun. 63 (1995) 2738-2742).

Les acides nucléiques selon l'invention et/ou les cassettes d'expression définies dans la demande peuvent être insérés dans un vecteur, qui constitue un autre objet de la présente invention. Avantageusement, il s'agit d'un vecteur fonctionnel dans les bactéries, c'est-à-dire capable de pénétrer dans les bactéries et d'y transporter les acides nucléiques de l'invention. Plus préférentiellement, un tel vecteur comporte soit une origine de réplication fonctionnelle dans une bactérie, soit des séquences lui permettant de s'intégrer dans le génome d'une bactérie. Il peut s'agir plus particulièrement d'un plasmide, phage, épisome, etc. En outre, certains vecteurs peuvent comporter avantageusement deux origines de réplication, l'une fonctionnelle dans des bactéries du genre E. coli, l'autre fonctionnelle dans des bactéries du type Clostridium par exemple.

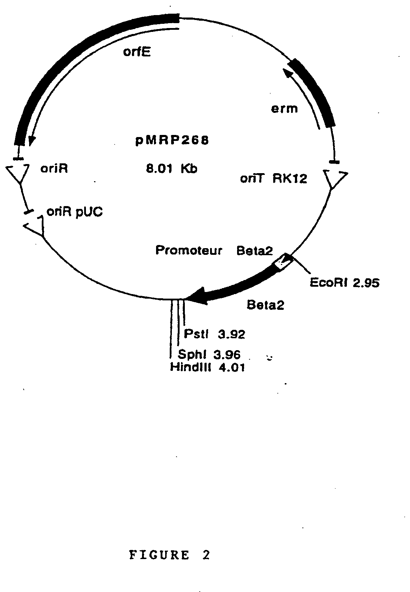

De manière particulièrement préférée, le vecteur de l'invention est un vecteur fonctionnel dans les bactéries du genre *Clostridium,* notamment dans les bactéries *Clostridium perfringens.* In peut s'agir à titre d'exemple d'un vecteur dérivé du plasmide pAT19 décrit par Trieu-Cuot et al. (Gene 102 (1991) 99-104). Un tel dérivé est par exemple le vecteur pMRP353 ou le vecteur pMRP268 tels que représentés sur les figures 2-4.

L'invention concerne en outre toute cellule recombinante comprenant un acide nucléique ou un vecteur de l'invention. Avantageusement, la cellule recombinante est une cellule procaryote, de préférence une bactérie. De manière particulièrement avantageuse, la cellule de l'invention est une bactérie du genre Clostridium choisie parmi C. absonum, C. baratii, C. bifermentans, C. chauvei, C. difficile, C. ghonii, C. lituseburense, C. novyi, C. perfringens, C. septicum, C. sordellii, C. subterminale et C. tetani. Encore plus préférentiellement, il s'agit d'une bactérie C. perfringens.

Les cellules recombinantes de l'invention peuvent être préparées en utilisant toutes les techniques connues de l'homme du métier permettant l'introduction d'un acide nucléique dans une cellule. Il peut s'agir par exemple de techniques physiques (électroporation, bombardement, "gene gun", etc.) de techniques chimiques (précipitation au CaPO3, utilisation d'agents de transfert chimiques : lipides cationiques, polymères, etc) ou d'autres méthodes telles que la fusion de cellules, la conjugaison, etc. (Scott et al., Gene 82 (1989) 327-333; Phillips-Jones, FEMS Microbiol. Letters 66 (1990) 221-226; Allen et al., FEMS, Microbiol. Letters 70 (1990) 217-220). L'invention concerne également un procédé de production d'un polypeptide comprenant l'introduction dans une cellule hôte d'un acide nucléique de l'invention codant pour ledit polypeptide sous contrôle d'un promoteur, puis la récupération dudit polypeptide.

Un procédé particulier de production de polypeptides selon l'invention comprend la culture d'une cellule recombinante telle que définie ci-dessus comprenant, le transgène codant pour ledit polypeptide.

Plus particulièrement, dans le procédé de l'invention, la cellule est une bactérie du genre Clostridium, encore plus préférentiellement C. perfringens.

La demande décrit la production d'une toxine ou d'un toxoïde. Le terme "toxoïde" est bien connu de l'homme du métier, et désigne toute forme inactivée d'une toxine, c'est-à-dire dépourvue de caractère toxique, mais conservant des propriétés immunologiques de la toxine. Le procédé de l'invention est utilisé pour la production de la toxine βB₂ de C. perfringens, selon la revendication 11.

De manière plus générale, l'invention concerne donc l'utilisation d'un acide nucléique de l'invention pour la production de polypeptides.

La demande décrit également un procédé de préparation d'une composition immunogène comprenant les étapes suivantes :
a) l'expression d'une ou plusieurs toxines (ou toxoïdes correspondants) dans une cellule telle de l'invention,
b) la récolte du surnageant,
c) de manière facultative, le traitement du surnageant pour purifier ou concentrer la (les) toxine(s) ou toxoïde(s),
d) l'inactivation de la (des) toxine(s), et
e) de manière facultative, le conditionnement de la (des) toxine(s) inactivée(s) ou du (des) toxoïde(s).

Dans ce procédé, une étape supplémentaire facultative, située avant ou après l'étape d), comprend le regroupement du surnageant avec d'autre(s) surganeant(s) de culture contenant une toxine différente ou identique, ou un toxoïde correspondant. Ce ou ces autres sumageants peuvent provenir de souches recombinantes telles que définies dans l'invention, ou de toute autre souche, recombinante ou non, productrice de la toxine considérée. Avantageusement, dans ce procédé deux surnageants sont regroupés, provenant de cultures de bactéries recombinantes selon l'invention produisant une toxine différente.

### a) Production des surnageants

La première étape dans la production des compositions immunogènes consiste à produire des surnageants de culture contenant la ou les toxines considérées. Avantageusement, les surnageants proviennent au moins en partie d'une souche bactérienne recombinante comprenant un vecteur de l'invention. Dans le cas de vaccins polyvalents, plusieurs voire tous les surnageants peuvent être des surnageants de souches bactériennes recombinantes comprenant un vecteur de l'invention. Plus préférentiellement, il s'agit de souches recombinantes de Clostridium, plus préférentiellement C. perfringens. Un des avantages du procédé est qu'il permet de limiter le nombre d'organismes différents employés pour les fermentations. Par ailleurs, les souches recombinantes utilisées peuvent également comprendre vecteurs de l'invention, de sorte qu'elles produisent simultanément plusieurs toxines. Ce mode de réalisation permet avantageusement de réduire en plus le nombre de fermentations dans le procédé de fabrication.

La production peut être réalisée dans les conditions de culture décrites ci-avant, en fermenteurs allant de 50 à 1500 litres. Lorsque les surnageants contenant les toxines ont été produits, ils sont soumis à différents traitements.

### b) Récolte des surnageants

Les surnageants sont récoltés par les techniques classiques de la biologie, bien connues de l'homme du métier. En particulier, les surnageants peuvent être récupérés par simple filtration permettant de séparer les cellules.

### c) Traitement des surnageants

De manière facultative, pour améliorer la qualité de la préparation finale, il est possible de soumettre les surnageants à des traitements supplémentaires tels que filtrations, centrifugations, concentrations, etc. Ces traitement permettent de clarifier les surnageants, et d'effectuer une purification partielle des toxines. Ces techniques sont connues de l'homme du métier. Par ailleurs, dans le cas de vaccins polyvalents, différents surnageants peuvent être regroupés à ce stade.

### d) Inactivation des toxines

Pour préparer une préparation immunogène efficace, il est bien entendu important que l'antigène utilisé soit dépourvu de la toxicité de la toxine contre laquelle une protection est recherchée. Dans le but de générer ces toxines inactivées (toxoïdes), différentes techniques sont envisageables (Rappuoli et al., Int. Arch. Allergy Immunol. 108 (1995) 327-333).

### Inactivation chimique

L'inactivation de toxines par voie chimique a été décrite depuis assez longtemps, et continue d'être utilisée aujourd'hui pour de nombreuses préparations immunogènes. Ainsi, dans le but d'inactiver les toxines bactériennes sans affecter trop leurs propriétés immunogènes, les surnageants peuvent être traités par les composés suivants : formol, β-propiolactone, iode, formaldéhyde ou glutaraldéhyde. Les conditions et doses plus précises utilsables sont connues de l'homme du métier, et ont été décrites par exemple par Rappuoli R. (In Woodrow GC, Levine MM (eds): New Generation Vaccines. New York, Dekker, 1990 p.251-268), incorporée à la présente par référence.

### Inactivation physique

L'inactivation des toxines peut également être réalisée par voir physique, par exemple par irradiation.

### . Inactivation par haute pression

L'inactivation peut encore être effectuée par traitement haute pression.

### . Inactivation génétique

Une autre approche pour l'inactivation des toxines repose sur la modification génétique de leur structure primaire. Dans ce cas, les produits libérés dans le surnageant sont déjà des toxoïdes, et il n'est pas nécessaire d'effectuer une étape supplémentaire d'inactivation. L'inactivation (ou détoxification) génétique consiste essentiellement à modifier les acides nucléiques codant pour les toxines, de sorte que un ou plusieurs acides aminés sont changés et en ce que la protéine produite soit dépourvue de toxicité. Par exemple, il est possible de remplacer des acides aminés impliqués dans l'activité enzymatique, et donc dans la toxicité, par des acides aminés différents dépourvus de cette activité. Il est également possible de déléter des régions de la toxine, de manière à fabriquer des fragments non-toxiques, immunogènes. Cette stratégie peut notamment être mise en oeuvre lorsque des épitopes des toxines ont été identifiés (par exemple par "epitope scanning") ou sont identifiables (par exemple à partir d'un profil d'hydrophobicité). Cette stratégie peut également être appliquée à la production de fragments (par exemple trypsiques) dont l'absence de toxicité a été mise en évidence. Par ailleurs, la modification génétique peut également être réalisée par mutagénèse au hasard, et sélection des clones produisant un toxoïde. Une telle stratégie a déja été mise en oeuvre avec succès pour produire des toxoïdes de la toxine diphtérique par mutagénèse au moyen de nitrosoguanidine (NTG) (Giannini et al., Nucleic Acids Res. 12 (1984) 4063-4069). Par ailleurs, la production de toxoïdes par mutagénèse site-spécifique a également été réalisée avec succès à partir des gènes des toxines pertussique et cholérique (Pizza et al., Science 246 (1989) 497-500 ; Pizza et al., J. Exp. Med. 6 (1994) 2147-2153 ; Fontana et al., Infect. Immun. 63 (1995) 2356-2360).

Les toxoïdes ainsi produits sont ensuite utilisés directement pour la préparation des compositions vaccinales.

### e) Formulation

Les toxoïdes sont généralement conditionnés selon les techniques classiques de la pharmacopée, de manière appropriée à un usage vaccinal. Préférentiellement, les toxoïdes sont formulés en présence d'adjuvants ou d'excipients, adaptés à la réalisation de solutés injectables. En particulier, l'injection est avantageusement réalisée par voie sous-cutanée ou systémique. Les doses d'antigène (toxoïde) utilisées sont généralement celles assurant la meilleure protection sans induire de réaction secondaire significative. Les conditions et sites d'injection, ainsi que les techniques de détermination des doses sont illustrées en détails dans la pharmacopée (Vaccinum Clostridii Perfringentis, Chap. 363).

La demande décrit également toute préparation immunogène comprenant une toxine produite dans une souche recombinante de l'invention. La demande décrit plus particulièrement toute préparation immunogène comprenant un toxoïde de la toxineBéta2 recombinante, éventuellement associée à d'autres toxoïdes.

La présente invention sera décrite plus en détails à l'aide des exemples qui suivent, qui doivent être considérés comme illustratifs et non limitatifs.

### Légende des Figures

- Figure 1 :: Stratégie de clonage du gène complet de la toxine Béta2 de C. perfringens. H, HindIII ; S, Sau3A.
- Figure 2 :: Représentation schématique du vecteur pMRP268
- Figure 3 :: Représentation schématique du vecteur pMRP353
- Figure 4 :: Représentation schématique d'un plasmide portant le promoteur Béta2
- Figure 5 :: Analyse en SDS-PAGE et Western Blot de la toxine Béta2 recombinante purifiée : (A) Gel SDS (0,1%)-PAGE (10%) coloré au bleu de coomassie de la toxine recombinante Béta2 (4,5 µg); (B) Western blot correspondant obtenu avec des anticorps anti-béta2.
- Figure 6 :: Etude de la toxicité de la toxine Béta2 purifiée sur les cellules I407. Photos prises au microscope à contraste de phases de cellules I407 controle (A) ou traitées par 20 µg/ml de toxine Béta2 (C) pendant 18 heures. (B) et (D) : visualisation du cytosquelette d'actine.
- Figure 7 :: Sensibilité de la toxine Béta2 à la trypsine. La toxine Béta2 (165 µg/ml) a été incubée sans (ligne 7) ou avec 16 µg/ml (ligne 1), 160 µg/ml (ligne 2), 400 µg/ml (ligne 3), 1,6 µg/ml (ligne 4), 4 µg/ml (ligne 5) et 16 µg/ml (ligne 6) de trypsine.
- Figure 8 :: Profil d'hydrophobicité de la toxine Béta2.

### Liste des Séquences

SEQ ID n° 1 : Séquence du gène de la toxine Beta2 de Clostridium Perfringens type C
SEQ ID n° 2 : Séquence du promoteur du gène de la toxine Beta2 de Clostridium Perfringens type C
SEQ ID n° 3 : Séquence du signal de sécrétion du gène de la toxine Beta2 de Clostridium Perfringens type C
SEQ ID n° 4 : Séquence de l'amorce P318
SEQ ID n° 5 : Séquence de l'amorce P292

### Matériels et Méthodes

### 1. Souches et ADNs bactériens utilisés.

Les souches bactériennes utilisées sont mentionnées dans les Tableaux 1 et 2. Les souches de Clostridium ont été cultivées en présence de Trypticase (30 g/litre), extrait de levure (20 g/litre), glucose (5 g/litre) et cystéine-HCl (0,5 g/litre), pH 7,2 (milieu TGY) à 37°C en conditions anaérobies.
L'ADN total et l'ADN plasmidique de Clostridium ont été extraits et purifiés selon la technique décrite par Perelle et al. (Infect. Immun. 61 (1993) 5147-5156).
Les plasmides pUC19 et pUC18 (Appligene, Strasbourg, France) ont été utilisés pour les expériences de clonage dans E. coli TG1, et les vecteurs navettes pAT19 (Trieu-Cuot et al., Gene 102 (1991) 99-104) et pJIR750 (Bannan et Rood, Plasmid 229 (1993) 233-235) ont été utilisés pour les expériences de clonage et d'expression dans Clostridium, notamment dans Clostridium perfringens 667-76, une souche négative pour la lécithine.
2. Les oligonucléotides synthétiques et les expériences d'hybridation ont été réalisées dans les conditions décrites dans Perelle et al, 1993 (supra).
3. Les expériences d'amplification par PCR ont été réalisées dans un volume total de 100 µl en utilisant 100 ng d'ADN comme décrit précédemment (Perelle et al, 1993, supra).
4. Les ligatures et transformations ont été effectuées selon les protocoles standard de la biologie moléculaire (Sambrook et al., Molecular Cloning: a laboratory manual. Cold Spring Harbor Laboratory Press. Cold Spring Harbor, N.Y., 1989).
5. La purification de la toxine Beta2 et son microséquençage ont été réalisés selon le protocole décrit dans la demande internationale n° WO95/17521.
6. La cytotoxicité a été déterminée selon le test suivant : Les cellules intestinales 1407 (ATCC) ont été cultivées en milieu Dulbecco modifié (DMEM) supplémenté par 5% de sérum de veau fétal. Les cellules I407 ont été étalées sur des plaques de culture 96 puits (Falcon, Becton Dickinson) et cultivées pendant 24 heures à 37°C dans un incubateur à 5% CO2 pour obtenir des monocouches. Des dilutions en série d'un facteur 2 d'échantillons de volume final 100 µl ont été ajoutées aux monocouches. Les cellules ont été examinées après 18 heures d'incubation, pour détecter tout changement de morphologie. Le cytosquelette d'actine a été visualisé par immunofluorescence au moyen de phalloidine isothyocyanate fluorescente (1 µg/ml, Sigma) selon la technique décrite par Giry et al. (Infect. Immun. 63 (1995) 4063-4071).

### Exemples

### A - Clonage du gène complet de la toxine Beta2 de Clostridium Perfringens type C

Cet exemple décrit le clonage du gène complet de la toxine Béta2, c'est-à-dire incluant les signaux en 5' de régulation et d'adressage.

Un fragment de 676 pb comprenant essentiellement la région codant pour la forme mature de la protéine a été isolé par amplification en utilisant les amorces P279 et P280 déduites du microséquençage de la protéine (WO95/17521). Ce fragment ne comporte pas le gène complet, dans la mesure où aucun signal de régulation en 5' du gène n'est présent. En outre, l'existence de séquences d'adressage n'est pas divulguée ni déductible de ce fragment. Dans le but de cloner le gène complet, les inventeurs ont dans un premier temps utilisé ce fragment comme sonde pour isoler, par hybridation à partir d'une banque génomique, un fragment de taille supérieure comportant la région 5'. Aucune de ces expériences n'a cependant permis de détecter ni, a fortiori, d'obtenir un fragment correspondant, quelles que soient les conditions d'hybridation utilisées. Les inventeurs ont donc imaginé une stratégie différente pour tenter d'isoler un fragment portant les régions 5' du gène. A cet égard, les inventeurs ont dans un premier temps circularisé la matrice servant à l'amplification, et procédé à une amplification par PCR inverse sur l'ADN ainsi obtenu, en utilisant les amorces P292 et P318, dont la séquence est la suivante :
P318 : 5'-GAAATGTTTACAACTGTATTAATATCGTAG-3' (SEQ ID n° 4)
P292 : 5'-TCAAGTTTGTACATGGGATGATG-3' (SEQ ID n° 5)

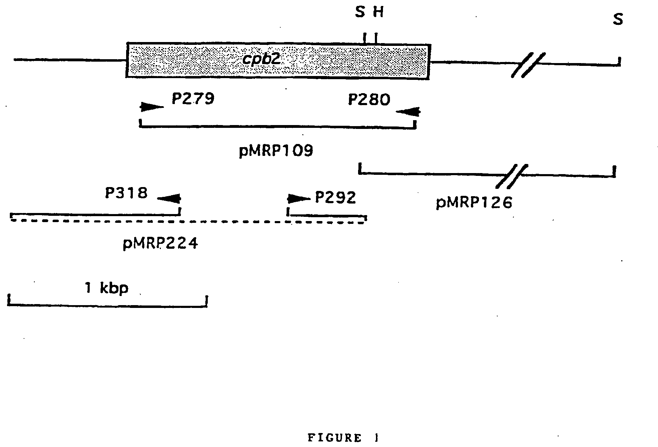

La localisation de ces amorces sur le gène est indiquée dans la séquence SEQ ID n°1. La stratégie de clonage est représentée sur la Figure 1. Le fragment amplifié ainsi obtenu a ensuite été sous cloné dans le plasmide pUC18 coupé par SmaI, pour générer le vecteur pMRP224 (Figure 1).

La séquence complète du fragment ainsi obtenu, de 1392 pb, est représentée sur la séquence SEQ ID n° 1.

### B - Identification des régions promotrices

La séquence obtenue dans l'exemple A (SEQ ID n° 1) comporte une phase ouverte de lecture, codant pour la toxine Béta2 mature (résidus 358 à 1062), et des régions régulatrices ou d'adressage situées en 5' et en 3'. La région promotrice du gène de la toxine Beta2 de Clostridium Perfringens type C peut être localisée sur cette séquence comme comprenant les résidus 1 à 267. Cette séquence du promoteur du gène de la toxine Beta2 de Clostridium Perfringens type C est présentée séparément sur la séquence SEQ ID n° 2. Cette séquence comporte notamment un site consensus de fixation des ribosomes (GGGGGG) localisé 7 nucléotides en amont du codon d'initiation ATG, soit aux positions 255-260 de la séquence SEQ ID n° 1.

Cette région, ou tout fragment ou variant de celle-ci, peut être isolée à partir d'échantillons d'acides nucléiques de clostridium en utilisant les sondes appropriées (par exemple correspondant à la séquence SEQ ID n° 2 ou un fragment de celle-ci), ou par synthèse chimique, ou par digestion enzymatique à partir des plasmides de l'invention, notamment du plasmide pMRP268, déposé le 8 août 1997 à la collection de l'Institut Pasteur (CNCM) sous le numéro I-1911.

L'activité de promoteur transcriptionnel de cette région ou des fragments ou variants peut être contrôlée de différentes manières, et notamment par insertion de cette région en amont d'un gène reporteur, et vérification de la présence du produit de transcription ou de traduction dudit gène reporteur dans un hote cellulaire approprié, notamment une bactérie du genre Clostridium, plus préférentiellement C. Perfringens.

Le gène reporteur peut être par exemple le gène LacZ ou encore le gène codant pour la luciférase.

La construction de telles cassettes d'expression ou vecteurs est illustrée dans les exemples D et suivants, ainsi que les conditions de transformation de différents hôtes cellulaires.

### C - Identification des régions d'adressage

La séquence obtenue dans l'exemple A (SEQ ID n° 1) comporte en plus d'une phase ouverte de lecture et de régions régulatrices de la transcription (exemple B) des signaux d'adressage permettant de diriger une protéine ou un peptide en cours de synthèse, vers les voies de sécrétion de la cellule hôte. La région d'adressage (peptide signal de sécrétion) du gène de la toxine Beta2 de Clostridium Perfringens type C peut être localisée sur la séquence SEQ ID n° 1 comme comprenant les résidus 268 à 357. Cette séquence du peptide signal du gène de la toxine Beta2 de Clostridium Perfringens type C est présentée séparément sur la séquence SEQ ID n° 3. Cette région code pour 30 acides aminés, comportant une région hydrophobe (résidus 6-26), formant vraisemblablement un domaine transmembranaire, bordée par des acides aminés chargés (Lys2, Lys3, Lys7 et Lys27). Par ailleurs, la région de jonction entre cette séquence signal et la protéine mature (AIa30-Lys31) correspond au site de clivage (Ala-X) de la majeure partie des signal peptidases bactériennes.

Cette région, ou tout fragment ou variant de celle-ci, peut être isolée à partir d'échantillons d'acides nucléiques de clostridium en utilisant les sondes appropriées (par exemple correspondant à la séquence SEQ ID n° 3 ou un fragment de celle-ci), ou par synthèse chimique, ou par digestion enzymatique à partir des plasmides de l'invention, notamment du plasmide pMRP268, déposé le 8 août 1997 à la collection de l'Institut Pasteur (CNCM) sous le numéro I-1911.

L'activité de séquence signal de cette région ou des fragments ou variants peut être contrôlée de différentes manières, et notamment par insertion de cette région en amont d'un gène reporteur, et vérification de la présence du produit de traduction dudit gène reporteur dans le surnageant de culture d'un hôte cellulaire approprié, notamment une bactérie du genre Clostridium, plus préférentiellement C. perfringens.

La construction de cassettes d'expression ou vecteurs comportant ce type de signal de sécrétion est illustrée dans les exemples qui suivent, ainsi que les conditions de transformation de différents hôtes cellulaires.

### D - Construction de cassettes d'expression et de vecteurs

Les régions de régulation et d'adressage décrites dans l'invention peuvent être insérées dans tout vecteur d'expression classique, ou utilisées pour la construction de cassettes d'expression.

Ces cassettes et vecteurs sont particulièrement adaptés à l'expression (et éventuellement la sécrétion) de protéines recombinantes dans des bactéries du genre Clostridium, notamment Clostridium perfringens. Il est entendu que tout autre type cellulaire dans lequel ces régions sont fonctionnelles peut être utilisé. Ces régions sont particulièrement avantageuses pour l'expression de toxines bactériennes, notamment de toxines de bactéries du genre Clostridium. La construction de cassettes et vecteurs appropriés est décrite ci-dessous.

### D1. Construction du vecteur pMRP268 (Figure 2)

Le vecteur pMRP268 porte les éléments suivants :
- une origine de réplication OriR permettant sa réplication dans une bactérie E. coli,
- une origine de réplication OriT permettant sa réplication dans une bactérie du genre Clostridium,
- deux gènes marqueur (orfE et erm), permettant la sélection des transformants chez E. coli et Clostridium,
- une cassette d'expression comportant, dans le sens 5'->3', le promoteur du gène de la toxine Béta2 de Clostridium perfringens, le signal de sécrétion du gène de la toxine Béta2 de Clostridium perfringens, et la séquence codant pour la toxine Béta2 de Clostridium perfringens.

Ce vecteur a été construit à partir du plasmide pAT19, par intoduction de la cassette d'expression au niveau du multisite de clonage. Plus particulièrement, la cassette a été obtenue par amplification du gène de la Béta2 de C. perfringens de séquence SEQ ID n° 1 au moyen des amorces P385 et P393, dont la séquence et la position sont représentées sur la séquence SEQ ID n° 1, en utilisant la Vent Polymerase (Biolabs) selon les recommandations du fabricant. Le produit d'amplification résultant a été inséré aux sites EcoRI-PstI du plasmide pAT19. La souche C. perfringens 667-76 n'exprimant pas la lécithine et contenant le plasmide pMRP268 a été déposée à la CNCM, Institut Pasteur, le 8 août 1997 sous la référence I-1911.

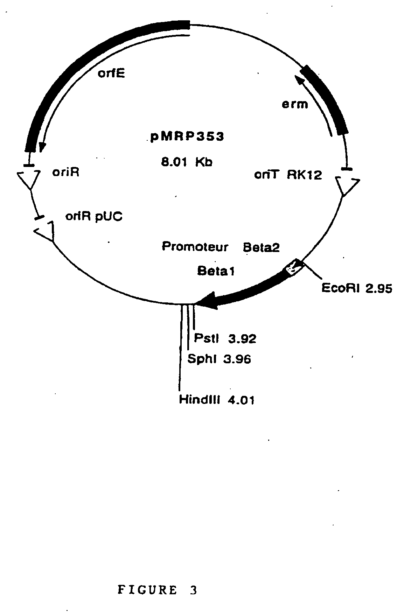

### D2. Construction du vecteur pMRP353 (Figure 3)

Le vecteur pMRP353 porte les éléments suivants :
- une origine de réplication OriR permettant sa réplication dans une bactérie E. coli,
- une origine de réplication OriT permettant sa réplication dans une bactérie du genre Clostridium,
- deux gènes marqueur (orfE et erm), permettant la sélection des transformants chez E. coli et Clostridium,
- une cassette d'expression comportant, dans le sens 5'->3', le promoteur du gène de la toxine Béta2 de Clostridium perfringens, le signal de sécrétion du gène de la toxine Béta2 de Clostridium perfringens, et la séquence codant pour la toxine Béta1 de Clostridium perfringens.

Ce vecteur a été construit à partir du plasmide pMRP268, par substitution de la séquence codant pour la toxine Béta2 de Clostridium perfringens par celle codant pour la toxine Béta1. L'ADNc codant pour la toxine Béta1 a été obtenu par amplification à partir de la souche NTCT8533 (Tableau 1) au moyen d'amorces introduisant un site Ncol à l'extrémité 5' (P321) et un site PstI à l'extrémité 3' (P322).

### D3. Construction d'un vecteur utilisable pour l'expression de tout ADNc

Il est entendu que les vecteurs décrits en D1 et D2 ci-dessus peuvent être utilisés pour l'expression de tout ADN d'intérêt sous le controle d'une région promotrice provenant du gène Béta2, par substitution de la séquence codante, comme illustré dans l'exemple D2. En outre, des vecteurs équivalents peuvent être construits à partir d'autres squelettes plasmidiques classiques, portant d'autres origines de réplications et marqueurs de sélection.

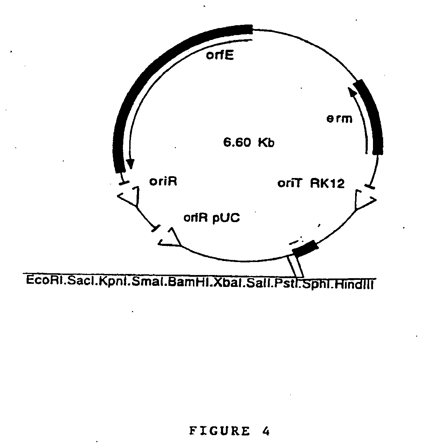

La Figure 4 représente un vecteur portant le promoteur du gène Béta2 de Clostridium perfringens, suivi d'un multisite de clonage permettant l'introduction de tout ADNc d'intérêt.

### E - Production et purification de la protéine Beta2 recombinante dans Clostridium.

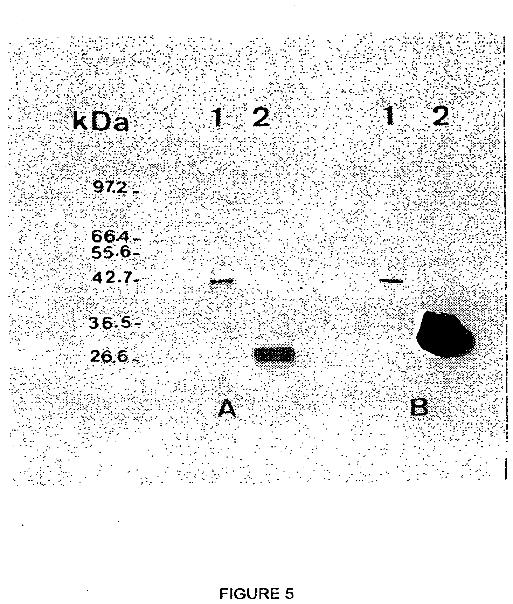

Le plasmide pMRP268 a été introduit dans la souche C. perfringens 667-76 par électroporation (Perelle et al., 1993). La souche recombinante a été cultivée pour une nuit dans du milieu TGY contenant 30 µg/ml d'érythromycine, en conditions anaérobies à 37°C. Le surnageant de culture a ensuite été précipité par saturation en présence de sulfate d'ammonium à 60%. Le précipité a été dialysé contre 10 mM de Tris-HCl, pH 7,5, et chargé sur une colonne de DEAE Sépharose CL6B. La colonne a ensuite été lavée et éluée en présence de NaCl 0,1 M dans le même tampon. Le matériel élué a été dialysé contre du PIPES-HCl 10 mM, pH 6,5, puis chargé sur une nouvelle colonne de DEAE Sépharose CL6B équilibrée avec le même tampon. Après lavage, la colonne a été éluée avec un gradient 0-0,1 M de NaCl dans un tampon PIPES. Les fractions contenant la toxine Béta2 recombinante purifiée ont été concentrées. Le poids moléculaire apparent de la protéine recombinante, déterminé par SDS-PAGE, est de 28 kDa, ce qui est en accord avec le poids moléculaire calculé d'après la séquence (Figure 5).

Les résultats obtenus montrent donc :
- qu'il est possible de produire et de sécréter une toxine recombinante dans Clostridium, sous contrôle du promoteur Béta2,
- que la toxine recombinante peut être purifiée,
- que la structure de la toxine recombinante ne semble pas altérée,
- que les niveaux d'expression obtenus sont supérieurs d'un facteur 10 à ceux observés dans une souche sauvage de Clostridium.

Une expérience a été réalisée dans les conditions silmilaires, mais en introduisant le vecteur d'expression non pas dans la souche 667-76 (qui ne produit pas la toxine Béta2), mais dans une souche sauvage de Clostridium. Les résultats obtenus montrent que la présence de vecteurs de l'invention dans les souches de Clostridium permet d'augmenter les niveaux de production de la toxine Béta2 d'un facteur 40 à 80.

Ces résultats témoignent des avantages fournis par la présente invention. Ainsi, la possibilité de produire, à des niveaux importants, dans une souche de Clostridium, différents types de toxines, permet d'améliorer le pouvoir immunogène de ces surnageants, d'élargir la gamme de vaccins à des toxines faiblement produites de manière naturelle, et de simplifier la production industrielle de compositions vaccinales.

### F - Production de la protéine Beta1 recombinante dans Clostridium.

Le plasmide pMRP353 a été introduit dans la souche C. perfringens 667-76 par électroporation (Perelle et al., 1993). La souche recombinante a été cultivée pour une nuit dans du milieu TGY contenant 30 µg/ml d'érythromycine, en conditions anaérobies à 37°C. Le surnageant de culture est traité comme dans l'exemple E.

Cette expérience permet de mettre en évidence la présence de Béta1 recombinante dans les surnageants.

Cet exemple illustre donc la capacité des constructions de l'invention à produire et à sécréter des toxines hétérologues (i.e. différentes de Béta2 ou provenant d'organismes pathogènes autres que C. perfringens) dans les souches de Clostridium.

### G - Production de compositions immunogènes

Comme indiqué ci-avant, la présente invention permet dorénavant de produire, à des niveaux importants, dans une souche de Clostridium, différents types de toxines pouvant entrer dans la compositions de vaccins. L'invention permet ainsi d'améliorer le pouvoir immunogène de ces vaccin, d'élargir la gamme des vaccins à des toxines faiblement produites de manière naturelle, et de simplifier la production industrielle de compositions vaccinales.

En particulier, la présente invention permet de produire des vaccins contenant un toxoïde de la toxine Béta2, c'est-à-dire une forme inactivée, permettant d'induire une protection améliorée contre les infections par Clostridium. Les avantages de telles compositions sont notamment illustrés par la démonstration des propiétés toxiques importantes de la toxine Béta2.

### G1. Propriétés de la toxine Beta 2 purifiée recombinante

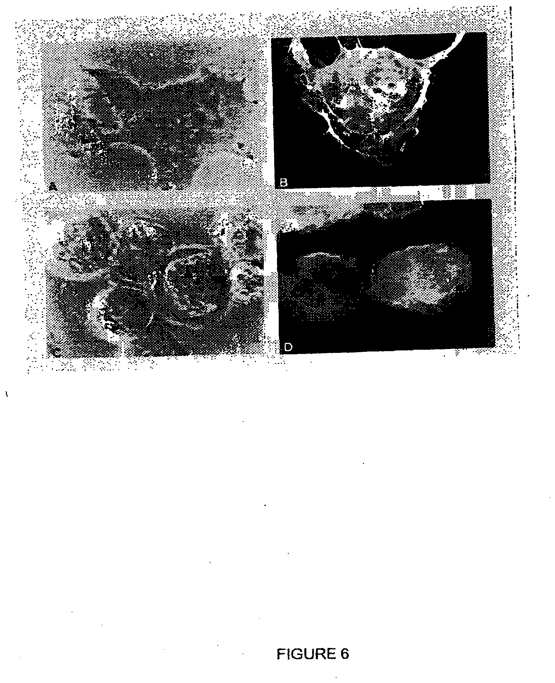

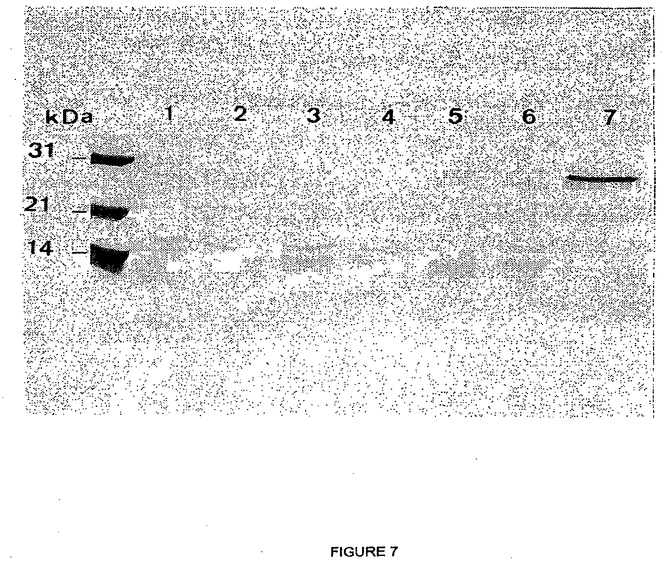

La toxine Béta2 purifiée a été injectée, par voie intraveineuse, à des souris. Les résultats obtenus montrent que cette toxine est léthale pour des souris, à des doses inférieures à 3 µg. En outre, les résultats présentés sur la Figure 6 montrent que la toxine Béta 2 est également toxique pour les cellules 1407.
Par ailleurs, l'effet d'un traitement de la béta2 par la trypsine sur l'activité de cette toxine a été évalué. Comme indiqué sur la Figure 7, la trypsine, à 16 ng/ml, clive la toxine béta2 en un constituant de 24 kDa, et à plus fortes concentrations, en deux peptides de 13 et 15 kDa. Les tests de cytotoxicité effectués avec ces produits de digestion trypsique montrent une absence totale de toxicité. De ce fait, la trypsine induit une perte de toxicité de la toxine béta2, et les peptides ainsi générés peuvent être utilisés comme toxoïdes.
Afin de déterminer l'importance de la toxine Béta2 dans la pathogénicité de Clostridium, la présence du gène correspondant a été analysée dans 57 souches de clostridium différentes (Tableaux 1 et 2). Les résultats obtenus montrent que certaines souches de Clostridium de type B et C portent le gène Béta2. En outre, parmi 27 souches isolées à partir de porcelets présentant des lésions de type nécroses à entérocolis, 44% portent le gène béta2. De plus, seul le gène Béta2 a été détecté dans toutes les souches isolées à partir de chevaux mourant des symptomes de colites et dans lesquels Clostridium perfringens a été récoltée en quantité élevée (supérieure à 10⁶/g) dans les extraits intestinaux. Ces résultats illustrent la corrélation entre la toxine béta2 et certaines pathologies animales, et l'intérêt de pouvoir générer des compositions vaccinantes dont l'un des antigènes est un toxoïde de la toxine béta2, notamment pour la vaccination des porcelets et des chevaux.

### G2. Production de compositions immunogènes

Cet exemple illustre la production de compositions immunogènes ou vaccinales destinées à protéger les organismes concernés des infections par des souches pathogènes bactériennes.

### a) Compositions polyvalentes ou monovalentes

Comme indiqué ci-avant, des compositions vaccinales peuvent être monovalentes (dirigées contre une seule toxine) ou polyvalentes (dirigées contre plusieurs toxines). Les vaccins disponibles sur le commerce sont généralement polyvalents (Miloxan, Gletvax5). Des compositions immunogènes préférées sont également polyvalentes.
Avantageusement, les compositions immunogènes comprennent au moins un toxoïde recombinant produit dans une cellule recombinante de l'invention. Une autre composition immunogène préférée comprend avantageusement un toxoïde de la toxine béta2 de C. perfringens. Les compositions immunogènes de l'invention peuvent comporter en outre toute toxine mentionnée précédemment.

### b) Production d'une composition immunogène contre la toxine Béta2.

Pour préparer une telle composition, le surnageant de culture de la souche C. perfringens transformée par le vecteur pMRP268 (exemple E) est récolté par les techniques classiques. Ce surnageant est centrifugé, puis filtré et concentré afin d'obtenir une préparation enrichie en toxines béta recombinantes. Cette préparation est ensuite soumise à un traitement par le formol afin d'inactiver les toxines présentes. L'efficacité du traitement est contrôlée par incubation de cellules I407 en présence d'un échantillon de cette préparation.

Cette préparation peut ensuite être utilisée comme immunogène pour induire, dans un organisme, la production d'anticorps. La capacité des anticorps produits à inhiber une infection par des souches pathogènes de Clostridium peut ensuite être déterminée, comme décrit dans la Pharmacopée (Vaccinum Clostridium Perfringens).

### c) Production d'une composition immunogène polyvalente

Pour préparer une telle composition, la préparation enrichie obtenue dans l'exemple b) ci-dessus est mélangée, avent ou après inactivation, avec un ou plusieurs autres surnageants de culture ou préparation dérivée comprenant une toxine ou un toxoïde correspondant, tel que par exemple un toxoïde pertussique, cholérique et/ou tétanique. La préparation résultante est ensuite contrôlée et utilisée comme décrit dans l'exemple b).

**TABLEAU 1**

| Souches de C. perfringens de Type C | Présence du gène de la toxine Beta2 |
|---|---|
| NCTC8533 | - |
| NCTC6121 | - |
| ATCC3628 | - |
| NCTC8081 | - |
| NCTC3180 | + |
| NCTC3182 | + |

**TABLEAU 2**

| Isolats de | Clostridium Totaux | Présence des gènes | | |
|---|---|---|---|---|
| | | cpb2+, cpb1- | cpb2+, cpb1 + | cpb2-, cpb1- |
| Porcelets | 27 | 12 | 12 | 1 |
| Chevaux | 15 | 16 | 0 | 0 |
| Aliments | 15 | 2 | 0 | 0 |
| cpb2 : Gène de la toxine béta2 ; cpb1 : Gène de la toxine béta1 | | | | |

Séquence SEQ ID n° 1

1 ATTTGGGATATCTTAAATTTAGCACAGAAGAATGTTTAAATGAAATAAAGATAATAAAAA
--------------------> P385
61 GATATATTAATTATATAGCTGAAAATTTATAATTATATGATAAGTATAGTTAATAAATAA
121 AAAGTGTTCTCGGGGACACTTTTTGTTTTAAAAAGGAAAATATAAATAAAATTTAGAT
181 AAAAGTGTAAAATAATTATTTTTATTTTAAATTTGTTAAAAATTTGATATAATTGAATTG
241 TAAAAAAAATTTCAGGGGGGAATATAAATGAAAAAAATTATTTCAAAGTTTACTGTAATT
1 M K K I I S K F T V I
301 TTTATGTTTTCATGTTTTCTTATTGTTGGAGCAATAAGTCCAATGAAAGCAAGTGCAAAA
12 F M F S C F L I V G A I S P M K A S A K
361 GAAATCGACGCTTATAGAAAGGTAATGGAGAATTATCTTAATGCTTTAAAAAACTACGAT
32 E I D A Y R K V M E N Y L N A L K N Y D
421 ATTAATACAGTTGTAAACATTTCAGAAGATGAAAGAGTAAATAATGTTGAACAGTATAGA
52 I N T V V N I S E D E R V N N V E Q Y R
481 GAAATGTTAGAAGATTTTAAATATGATCCTAACCAACAACTGAAATCTTTTGAAATACTT
72 E M L E D F K Y D P N Q Q L K S F E I L
541 AATTCACAAAAGAGCGATAATAAAGAAATATTTAATGTAAAAACTGAATTTTTAAATGGTO
92 N S Q K S D N K E I F N V K T E F L N G
601 GCAATTTATGATATGGAATTTACTGTATCATCTAAAGATGGAAAATTAATAGTATCTGAT
112 A I Y D M E F T V S S K D G K L I V S D
661 ATGGAAAGAACAAAAGTTGAGAATGAAGGAAAATATATTTTAACACCATCATTTAGAACT
132 M E R T K V E N E G K Y I L T P S F R T
721 CAAGTTTGTACATGGGATGATGAACTAGCACAAGCAATTGGGGGAGTTTATCCACAAACA
152 Q V C T W D D E L A Q A I G G V Y P Q T
781 TATTCTGATAGATTTACATATTATGCAGATAATATATTATTAAACTTCAGACAATATGCA
172 Y S D R F T Y Y A D N I L L N F R Q Y A
841 ACTTCAGGTTCAAGAGATTTAAAAGTAGAATATAGTGTTGTAGATCATTGGATGTGGAAA
192 T S G S R D L K V E Y S V V D H W M W K
901 GATGATGTTAAAGCTTCTCAAATGGTATATGGTCAAAATCCTGATTCTGCTAGACAAATA
212 D D V K A S Q M V Y G Q N P D S A R Q I
961 AGATTATATATAGAAAAAGGACAATCTTTCTATAAATATAGAATAAGAATTAAAAACTTT
232 R L Y I E K G Q S F Y K Y R I R I K N F
1021 ACACCTGCATCAATTAGAGTATTTGGTGAAGGTATTGTGCATAGAAAAAAATATGAAGT
252 T P A S I R V F G E G Y C A * <---------------
1081 GACTTAGTCACTTCATATTTTTTTTACTATTAATTTTATTATATAAAAACCTAACATACA
--- ----------------->
1141 TGAAAGTATTCTTAATACAGTTATATCAAAATTAAAGTAGGGGAAATAAAATAAAAGGCT
1201 AAAAACTATATTAAAAACTATAAAAATTATTAAATTAGGTTTTAAGGTGTTATATTTATT
1261 TATGATTATAGGAATAAATATGCCAAATGGAATAAATAAAAGTAATATTAATAATTGGTC
1321 TAAAAAGTATACATCATTGATAAAAGAAAAATTACCAGTAAAAATTGAGCTTAAAAAATT
P393 <-----------------------
1381 AAATGTAAATTT 1392

Séquence SEQ ID n° 2

1 ATTTGGGATATCTTAAATTTAGCACAGAAGAATGTTTAAATGAAATAAAGATAATAAAAA
-----------------------> P385
61 GATATATTAATTATATAGCTGAAAATTTATAATTATATGATAAGTATAGTTAATAAATAA
121 AAAGTGTTCTCGGGGACACTTTTTTGTTTTAAAAAGGAAAATATAAATAAAATTTAGAT
181 AAAGTGTAAAATAATTATTTTTATTTTAAATTTGTTAAAAATTTGATATAATTGAATTG
241 TAAAAAAAATTTCAGGGGGGAATATAA

Séquence SEQ ID n° 3

ATGAAAAAAATTATTTCAAAGTTTACTGTAATT
M K K I I S K F T V I
TTTATGTTTTCATGTTTTCTTATTGTTGGAGCAATAAGTCCAATGAAAGCAAGTGCA
F M F S C F L I V G A I S P M K A S A

Séquence SEQ ID n° 4
   P318: 5'-GAAATGTTTACAACTGTATTAATATCGTAG-3'
Séquence SEQ ID n° 5
   P292: 5'-TCAAGTTTGTACATGGGATGATG-3'

## Revendications

1. Acide nucléique choisi dans le groupe constitué par :
a) un acide nucléique, codant pour la toxine β2 mature et sa séquence d'adressage de *Clostridium perfringens,* tel que défini à la SEQ ID NO:1 ;
b) un acide nucléique, codant pour un variant de la toxine β2 mature et sa séquence d'adressage telle que définie à la SEQ ID NO :1, ladite toxine β2 mature du variant présentant des mutations de ses résidus d'acides aminés par rapport à la SEQ ID NO :1, ledit variant conservant la capacité à être sécrété.

2. Acide nucléique selon la revendication 1, codant pour un variant de la toxine β2 mature et sa séquence d'adressage telle que définie à la SEQ ID NO :1, ladite toxine β2 mature présentant 1 à 10 mutations de ses résidus d'acides aminés par rapport à la SEQ ID NO :1, ledit variant conservant la capacité à être sécrété.

3. Acide nucléique selon la revendication 1 ou 2 caractérisé en ce la toxine β2 mature qu'il code est génétiquement inactivée.

4. Vecteur comprenant un acide nucléique selon l'une quelconque des revendications 1 à 3.

5. Vecteur selon la revendication 4 **caractérisé en ce qu'**il est fonctionnel dans les bactéries.

6. Vecteur selon la revendication 5 **caractérisé en ce qu'**il est fonctionnel dans les bactéries du genre *Clostridium,* notamment dans les bactéries *Clostridium perfringens.*

7. Vecteur selon l'une quelconque des revendications 4 à 6, **caractérisé en ce qu'**il s'agit du plasmide pMRP268 déposé à la CNCM, le 8 août 1997, sous la référence I-1911.

8. Cellule recombinante comprenant un acide nucléique selon l'une quelconque des revendications 1 à 3, ou un vecteur selon l'une quelconque des revendications 4 à 7.

9. Cellule selon la revendication 8 **caractérisée en ce qu'**il s'agit d'une cellule procaryote.

10. Cellule selon la revendication 9 **caractérisée en ce qu'**il s'agit d'une bactérie.

11. Procédé de production d'une toxine β2 mature et sa séquence d'adressage, comprenant l'introduction dans une cellule hôte d'un acide nucléique selon l'une quelconque des revendications 1 à 3 sous le contrôle d'un promoteur, puis la récupération de ladite toxine β2 mature avec sa séquence d'adressage.

12. Procédé de production d'une toxine β2 mature et sa séquence d'adressage selon la revendication 11, **caractérisé en ce qu'**il comprend la culture d'une cellule recombinante selon l'une quelconque des revendications 8 à 10.

13. Procédé selon la revendication 12 **caractérisé en ce que** la cellule est une bactérie du genre *Clostridium.*

14. Utilisation d'un acide nucléique selon l'une quelconque des revendications 1 à 3 pour la production d'une toxine β2 mature et sa séquence d'adressage.

## Patentansprüche

1. Nukleinsäure ausgewählt aus der Gruppe bestehend aus:
a) einer Nukleinsäure, kodierend für das maturierte Toxin β2 und seine Zielsequenz von *Clostridium perfringens* wie in SEQ ID NO:1 definiert,
b) eine Nukleinsäure, kodierend für eine Variante des maturierten Toxins β2 und seine Zielsequenz wie in SEQ ID NO:1 definiert, wobei das maturierte Toxin β2 der Variante an seinen Aminosäureresten Mutationen gegenüber SEQ ID NO:1 aufweist, wobei die Variante die Fähigkeit, sezerniert zu werden, beibehält.

2. Nukleinsäure nach Anspruch 1, kodierend für eine Variante des maturierten Toxins β2 und seine Zielsequenz wie in SEQ ID NO:1 definiert, wobei das maturierte Toxin β2 an seinen Aminosäureresten 1 bis 10 Mutationen gegenüber SEQ ID NO:1 aufweist, wobei die Variante die Fähigkeit, sezerniert zu werden, beibehält.

3. Nukleinsäure nach Anspruch 1 oder 2, **dadurch gekennzeichnet, dass** das maturierte Toxin β2, das sie kodiert, genetisch inaktiviert ist.

4. Vektor, umfassend eine Nukleinsäure nach einem beliebigen der Ansprüche 1 bis 3.

5. Vektor nach Anspruch 4, **dadurch gekennzeichnet, dass** er in Bakterien funktionell ist.

6. Vektor nach Anspruch 5, **dadurch gekennzeichnet, dass** er in Bakterien der Gattung *Clostridium,* insbesondere in den Bakterien *Clostridium perfringens* funktionell ist.

7. Vektor nach einem beliebigen der Ansprüche 4 bis 6, **dadurch gekennzeichnet, dass** es sich um das Plasmid pMRP268 handelt, hinterlegt bei der CNCM am 8. August 1997 unter der Referenz I-1911.

8. Rekombinante Zelle, umfassend eine Nukleinsäure nach einem beliebigen der Ansprüche 1 bis 3 oder einen Vektor nach einem beliebigen der Ansprüche 4 bis 7.

9. Zelle nach Anspruch 8, **dadurch gekennzeichnet, dass** es sich um eine prokaryotische Zelle handelt.

10. Zelle nach Anspruch 9, **dadurch gekennzeichnet, dass** es sich um ein Bakterium handelt.

11. Verfahren zur Herstellung eines maturierten Toxins β2 und seiner Zielsequenz, umfassend das Einführen einer Nukleinsäure nach einem beliebigen der Ansprüche 1 bis 3 in eine Wirtszelle unter Kontrolle eines Promotors, danach Rückgewinnen des maturierten Toxins β2 mit seiner Zielsequenz.

12. Verfahren zur Herstellung eines maturierten Toxins β2 und seiner Zielsequenz nach Anspruch 11, **dadurch gekennzeichnet, dass** es die Kultur einer rekombinanten Zelle nach einem beliebigen der Ansprüche 8 bis 10 umfasst.

13. Verfahren nach Anspruch 12, **dadurch gekennzeichnet, dass** die Zelle ein Bakterium der Gattung *Clostridium* ist.

14. Verwendung einer Nukleinsäure nach einem beliebigen der Ansprüche 1 bis 3 für die Herstellung eines maturierten Toxins β2 und seiner Zielsequenz.

## Claims

1. A nucleic acid selected from the group constituted by:
a) a nucleic acid coding for the mature β2 toxin and its addressing sequence of *Clostridium perfringens* as defined in SEQ ID NO: 1;
b) a nucleic acid coding for a variant of the mature β2 toxin and its addressing sequence as defined in SEQ ID NO: 1, said mature β2 toxin of the variant exhibiting mutations of its amino acid residues compared with SEQ ID NO: 1, said variant conserving the ability to be secreted.

2. The nucleic acid as claimed in claim 1, coding for a variant of the mature β2 toxin and its addressing sequence as defined in SEQ ID NO: 1, said mature β2 toxin having 1 to 10 mutations of its amino acid residues compared with SEQ ID NO: 1, said variant conserving the ability to be secreted.

3. The nucleic acid as claimed in claim 1 or claim 2, **characterized in that** the mature β2 toxin for which it codes is genetically inactivated.

4. A vector comprising a nucleic acid as claimed in any one of claims 1 to 3.

5. The vector as claimed in claim 4, **characterized in that** it is functional in bacteria.

6. The vector as claimed in claim 5, **characterized in that** it is functional in bacteria of the genus *Clostridium,* in particular in *Clostridium perfringens* bacteria.

7. The vector as claimed in any one of claims 4 to 6, **characterized in that** it is the plasmid pMRP268 deposited on 8th August 1997 with the CNCM with accession number I-1911.

8. A recombinant cell comprising a nucleic acid as claimed in any one of claims 1 to 3, or a vector as claimed in any one of claims 4 to 7.

9. The cell as claimed in claim 8, **characterized in that** it is a prokaryotic cell.

10. The cell as claimed in claim 9, **characterized in that** it is a bacterium.

11. A process for producing a mature β2 toxin and its addressing sequence, comprising introducing a nucleic acid as claimed in any one of claims 1 to 3 into a host cell under the control of a promoter, then recovering said mature β2 toxin with its addressing sequence.

12. The process for producing a mature β2 toxin and its addressing sequence as claimed in claim 11, **characterized in that** it comprises culturing a recombinant cell as claimed in any one of claims 8 to 10.

13. The process as claimed in claim 12, **characterized in that** the cell is a bacterium of the genus *Clostridium.*

14. Use of a nucleic acid as claimed in any one of claims 1 to 3, for the production of a mature β2 toxin and its addressing sequence.
